# EUROPEAN PATENT APPLICATION

(11) **EP 0 836 851 A1**
(43) Date of publication of application: **22.04.1998**
(21) Application number: 96402234.7
(22) Date of filing: 21.10.1996
(51) Int. Cl.: A61K 31/44

(54) **Amidine compounds for use in treating ecto or endo parasitic diseases and systemic parasite control compositions**

(71) Applicant: VIRBAC S.A., F-06516 Carros Cedex (FR)
(72) Inventor: Derrieu, Guy, 06800 Cagnes sur Mer (FR); Beuvry, Vincent, 06200 Nice (FR); Gozlan, Thierry, 06400 Cannes (FR)
(74) Representative: Orès, Bernard

(57) **Abstract**

The invention provides in compounds and compositions for killing both ecto and endo parasites which comprises administering to a warm blooded animal at least a systemically effective amount of an amidine of the formula I: 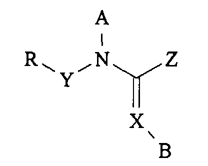
wherein R represents an optionally substituted 5 or 6 member aromatic ring containing at least one nitrogen atom;
Y represents an optionally substituted C₁-C₃ alkylene or alkylidene;
A represents a hydrogen, a carbamoyl, a mono or di C₁₋₅ alkyl carbamoyl, a thiocarbamoyl, a mono or di C₁₋₅ alkylthiocarbamoyl, a sulfamoyl, a mono or di C₁₋₅ alkylsulfamoyl, an optionally substituted C₁₋₅ alkyl, an optionally substituted C₂₋₅ alkenyl, an optionally substituted C₂₋₅ alkynyl, an optionally substituted C₃₋₈ cycloalkyl, an optionally substituted C₃₋₈ cycloalkenyl, an optionally substituted aryl or -D-R₂, where D is O, S(O)_{n'} CO, CS or CO₂ ; n is 0, 1, or 2, and R₂ is a hydrogen, an optionally substituted C₁₋₅ alkyl, an optionally substituted C₂₋₅ alkenyl, an optionally substituted C₂₋₅ alkynyl, an optionally substituted C₃₋₈ cycloalkyl, an optionally substituted C₃₋₈ cycloalkenyl, an optionally substituted aryl;
Z represents a hydrogen, an optionally substituted C₁₋₅ alkyl, an optionally substituted C₂₋₅ alkenyl, an optionally substituted C₂₋₅ alkynyl, an optionally substituted C₃₋₈ cycloalkyl, an optionally substituted C₃₋₈ cycloalkenyl;
B represents a cyano or a nitro; and
X represents CH or N; or its salt.

## Description

The present invention relates to amidine compounds for use in treating ecto and/or endo parasitic diseases in warm blooded animals and to systemic parasite control compositions containing said compounds.

Said amidine compounds have the generic following structure (formula I): 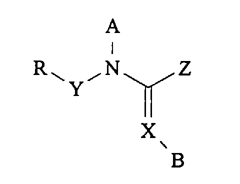
wherein R represents an optionally substituted 5 or 6 member aromatic ring containing at least one nitrogen atom;
Y represents an optionally substituted C₁-C₃ alkylene or alkylidene;
A represents a hydrogen, a carbamoyl, a mono or di C₁₋₅ alkyl carbamoyl, a thiocarbamoyl, a mono or di C₁₋₅ alkylthiocarbamoyl, a sulfamoyl, a mono or di C₁₋₅ alkylsulfamoyl, an optionally substituted C₁₋₅ alkyl, an optionally substituted C₂₋₅ alkenyl, an optionally substituted C₂₋₅ alkynyl, an optionally substituted C₃₋₈ cycloalkyl, an optionally substituted C₃₋₈ cycloalkenyl, an optionally substituted aryl or -D-R₂, where D is O, S(O)_{n'} CO, CS or CO₂; n is 0, 1, or 2, and R₂ is a hydrogen, an optionally substituted C₁₋₅ alkyl, an optionally substituted C₂₋₅ alkenyl, an optionally substituted C₂₋₅ alkynyl, an optionally substituted C₃₋₈ cycloalkyl, an optionally substituted C₃₋₈ cycloalkenyl, an optionally substituted aryl;
Z represents a hydrogen, an optionally substituted C₁₋₅ alkyl, an optionally substituted C₂₋₅ alkenyl, an optionally substituted C₂₋₅ alkynyl, an optionally substituted C₃₋₈ cycloalkyl, an optionally substituted C₃₋₈ cycloalkenyl;
B represents a cyano or a nitro; and
X represents CH or N; or its salt.

Said amidine compounds are known to be biologically active as agonists of the acetylcholine nicotinate receptor. Said compounds exhibit insecticidal activity levels against plant feeding insects and have only been used in agriculture. Members of the group are also active against fleas, lice, flies, and certain ticks.

The compound of the structure 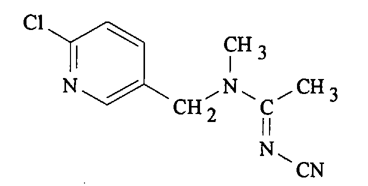 is known as an agrochemical bioregulator.

The Patent US 5 304 566 describes, for instance, the use of the hereabove mentioned compounds of formula I as agricultural chemicals; such compounds have a high insecticidal activity against both hemipterous and lepidopterous insects.

To control insect development in agriculture, other compounds, compositions or methods have been proposed. For instance, JP 05155707 describes slow release agricultural compositions containing N-cyano-N'-(2-chloro-5-pyridylmethyl)-N'-methylacetamidine, which are useful as insecticides for soil application; DE 4 409 039 describes a composition having an anti-fouling activity comprising a benzoylurea or a nitroimine insecticide; EP 627 165 describes a composition for the extermination of cockroach comprising a chemical insecticide selected from nitroguanidines and nitromethylenes and an entomopathogenic fungus; JP 06336406 obtains a good insecticidal activity by association of a hydrazine derivative with N'-[(6-chloro-3-pyridyl)methyll-N''-cyano-N'-methylacetamidine and/or toxin of *Bacillus thuringiensis*.

Non-systemic use of amidines, essentially bicyclic compounds, on animals had been described in the Patent Application EP 682 869; said non-systemic ectoparasiticidal activity is related to their nicotinergic acetylcholine receptor activity; such compounds are efficient to control fleas, lice, flies.

Systemic activity of numerous products have been described: certain insecticidal and ovicidal compounds are known to exert systemic activity in warm blooded animals. For example Miller, U.S. Patent No 5,439,924, discloses systemic use of ovocidal compounds.

Systemic agents directed against ectoparasites are known. For example, U.S. Pat. Nos 3,962,458 and 4,031,239 disclose the application of various cyclopropane carboxylate compounds (pyrethroids) for controlling ectoparisites by systemic treatment of warm blooded animals. U.S. Pat. No 4,006,236 of American Cyanamide discloses the systemic use of substituted octahydrophenanthridines. U.S. Pat. No 4,053,631 discloses the systemic control of ectoparasites with alpha-cyano-m-phenoxybenzylalpha -C₁-C₄ alkyl-2-naphthaleneacetates with a low mammalian toxicity.

Using as systemic agents the classes of methoprene-like juvenile hormones, flea growth inhibiting benzoylurea derivatives, and flea growth inhibiting triazine derivatives is disclosed in U.S. Pat. No 4,973,589. Systemically active compounds effective against endoparasites include avermectins, avermectin-like derivatives and a group of related antibiotics, for instance milbemycin, milbemycin-like derivatives, ivermectin, ivermectin-like-derivatives, milbemycin oxime, milbemycin oxime-like derivatives, moxidectin, moxidectin-like derivatives, and mixtures thereof, as disclosed by U.S. Pat. Nos. 3,950,360, (milbemycin), 4,199,569 (ivermectin), 4,547,520 (milbemycin oxime) and 4,988,824.

Insects develop resistance to insecticidal materials and new systemic compounds are continually needed to replace older compounds. Systemic activity is the result of a complex series of factors when a compound is administered to a warm blooded animal, absorbed in the body fluids or tissues such as skin, and retains biological activity when fed by a parasite. Scientific prediction of systemic activity after an administration to warm blood animal, based on chemical structure or direct contact biological activity is not currently possible.

Therefore, it is an aim of the present invention to provide alternative compositions for the treatment of ecto and/or endo parasitic diseases in warm blooded animals.

Unexpectedly, it has now been found that amidines of the formula I: 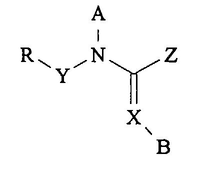
wherein R represents an optionally substituted 5 or 6 member aromatic ring containing at least one nitrogen atom;
Y represents an optionally substituted C₁-C₃ alkylene or alkylidene;
A represents a hydrogen, a carbamoyl, a mono or di C₁₋₅ alkyl carbamoyl, a thiocarbamoyl, a mono or di C₁₋₅ alkylthiocarbamoyl, a sulfamoyl, a mono or di C₁₋₅ alkylsulfamoyl, an optionally substituted C₁₋₅ alkyl, an optionally substituted C₂₋₅ alkenyl, an optionally substituted C₂₋₅ alkynyl, an optionally substituted C₃₋₈ cycloalkyl, an optionally substituted C₃₋₈ cycloalkenyl, an optionally substituted aryl or -D-R₂, where D is O, S(O)_{n'} CO, CS or CO₂ ; n is 0, 1, or 2, and R₂ is a hydrogen, an optionally substituted C₁₋₅ alkyl, an optionally substituted C₂₋₅ alkenyl, an optionally substituted C₂₋₅ alkynyl, an optionally substituted C₃₋₈ cycloalkyl, an optionally substituted C₃₋₈ cycloalkenyl, an optionally substituted aryl;
Z represents a hydrogen, an optionally substituted C₁₋₅ alkyl, an optionally substituted C₂₋₅ alkenyl, an optionally substituted C₂₋₅ alkynyl, an optionally substituted C₃₋₈ cycloalkyl, an optionally substituted C₃₋₈ cycloalkenyl;
B represents a cyano or a nitro; and
X represents CH or N; or its salt are useful in treating by systemic administration, both ecto and/or endo parasitic diseases in warm blooded animals.

Since compounds of this general structure are known to be acetylcholine nicotinate agonists, the observed systemic effect after administration to the warm blood animal is quite unexpected.

Therefore, the present invention relates to said amidines for use in treating both ecto and endo parasitic diseases in warm blooded animals.

Said amidines are active but not toxic when administered to a warm blooded animal in a systemically effective amount, able to produce a systemically effective concentration of active ingredient.

In a preferred embodiment the compound is N-[(6-halo-3-pyridinyl)C₁₋₃ alkyl)]-N'-cyano-N-C₁₋₃ alkylethaneimidamide, and especially preferred is a compound of the structure : 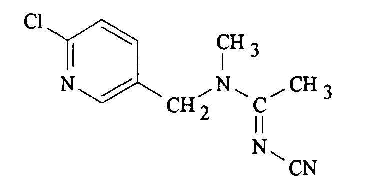 named as N-[(6-chloro-3-pyridinyl)methyl)]-N-cyano-N-methylethane imidamide referred to below as 〈〈 acetamiprid 〉〉.

The compounds are administered in an effective dosage determined by standard dose titration methods by any convenient route of administration. Especially said amidine is administered orally, parenterally, topically, by implant or as a bolus, formulated with pharmaceutically acceptable carriers, adjuvants, extenders, and other ingredients, active or inert to achieve systemic control of parasites. Administration may be at regular hourly intervals, or one may employ sustained release methods to provide the desired dose over a prolonged period of time.
In the instant invention:
- systemic activity means that a compound administered to a warm blooded animal maintains biological activity at a level sufficient to be effective against a parasite, feeding on the animal to which the compound has been administered, without producing an unacceptably harmful effect on the animal.
- effective dose means a quantity of a substance administered to an animal to be protected against or treated for a parasitic infection which provides a sufficient quantity of biologically active compound in the animals body fluids or tissues such as skin, to deliver a biologically active dose to a target parasite, feeding on the animal. The effective dose will vary from animal to animal and from parasite to parasite; however the methodologies for determining effective doses are well established in the art, and can be readily determined by those skilled in the art for a particular target animal and parasite to be treated.

The instant invention also relates to compositions containing an effective parasiticidal amount of at least one amidine as defined hereabove in combination with at least one carrier, diluent or excipient therefor and eventually another parasiticidal compound, for systemic use in treating both ecto and endo parasitic diseases.

Said compositions are useful in treating ecto and endoparasitic diseases: parasites are exposed to an effective amount of the systemically active ingredient when they ingest body fluids or various tissue from an animal which has been treated with said systemic effective dose. In said compositions, the amidines may be administered in doses of about 0.0001 mg/kg to about 1000 mg/kg preferably about 0.01 mg/kg to about 100 mg/kg, more preferably about 0.01 mg/kg to about 70 mg/kg and most preferably about 0.02 to about 50 mg/kg. In said compositions, said amidines may also be effective at low dosage range of 0.00001 mg/kg to 2 mg/kg and in the range 0.0001 mg/kg to about 1 mg/kg. In an additional, preferred embodiment, in said compositions, said amidines in these amounts may be combined with other parasitic control agents such as, for example, an avermectin, avermectin-like derivatives, milbemycin, milbemycin derivatives, ivermectin, ivermectin derivatives, milbemycin oxime, milbemycin oxime derivatives, moxidectin, or moxidectin derivatives and mixtures thereof, in doses in the range of about 0.5 µg/kg to about 100 mg/kg. In said compositions, the amidines may also be combined with insecticides such as IGR (Inhibitor growth Regulator) like pyriproxyfen or IDI (Insects development Inhibitor) like phenoxybenzylethers.

It is an essential feature of the present invention that the active compound is administered in such a manner that it can be ingested by the feeding parasite along with the blood, body fluids or tissue such as skin of the host animal, and can then exhibit activity against the parasite. In accordance with the present invention, this is achieved by several forms of applications. For example, the instant compositions may be administered orally, parenterally, by implant, as a bolus, by a topical application method like pour-on or spot-on formulation, spray, lotion or shampoo formulation.

Said compositions may be in the form of a powder, a tablet, a wafer, a granule, a capsule, a solution, an emulsion, a paste, a gel, a foam, or other compositions suitable for administering an effective amount of the active amidine. The instant composition does not necessarily have to be administered to the animal directly; it may be convenient to mix it with the animal's feed. In addition to containing adjuvants conventionally employed in the art of formulation, the compositions to be administered orally may of course contain further additives which stimulate voluntary ingestion by the animal, such as suitable scents or flavourings.

Owing to its simplicity, topical application is one of the preferred modes of administration of the instant compositions. Further modes of application are oral or parenteral, for example, by subcutaneous, intravenous, or intramuscular injection, or by means of a sustained action preparation in the form of an implant, bolus or other sustained release formulations. The application may be in a multiple dose or a single dose form.

Topical application or administration is a particularly interesting one form of administration of the instant compositions: economically satisfactory and easy to apply by pour-on, spot-on, spray, lotion or shampoo formulation in a solvent or solvents combination adapted to a topical application for the animal. Preferably the topical carrier is selected from the group consisting of alcohols, polyalcohols, glycol ethers, propylene glycol ethers, formamide, dimethylformamide, dialkylsulfoxydes like, DMSO, dimethylacetamide, glycerol formal, fatty acids and fatty esters especially when transesterified.

Oral administration include, but is not limited to, amidine premixed in animal food, fed in biscuits or treats, chewable tablets or wafers, water dissolvable capsules or tablets, emulsifiable concentrates, water soluble compounds applied with a dropper into water, or materials applied in any form onto pet food.

Parenteral administration is conveniently accomplished by subcutaneous, intradermal, intramuscular, and even intravenous application of the injectable formulation. Conventional needle-type injection devices, as well as needleless air blast injection devices, as well as pour-on and post-on formulations may be useful. It is possible to delay or sustain the permeation of the active ingredient through the animal's living tissues by proper formulation.

Implants may include any device applied to the animal for release of compounds to control ectoparasites.

Sustained action of the active ingredient can be obtained by formulating the compound in a matrix that will physically inhibit dissolution. The formulated matrix is injected or otherwise surgically implanted into the body, where it remains as a depot from which the compound slowly dissolves, or in the case of hydrophobic compounds, is released by diffusion. Matrix formulations, known in the art, are formulated in waxy semi-solids such as vegetable waxes and high molecular weight polyethylene glycols. Very effective sustained action is obtained by introducing into the animal, an implant containing the active ingredient. Such implants are well known in the veterinary art and are usually made of a silicon rubber or other polymerised plastic such as methacrylate. An especially useful implant composition is disclosed in U.S. Pat No. 4,696,974. The active ingredient is dispersed through the solid implant or is contained inside a hollow implant. The active ingredient is dispersed by first dissolving or mixing with the polymer, or dissolved in, or mixed with a carrier, it is further dispersed within the polymer. After implantation, the active ingredient diffuses or leaches out of the solid or hollow implant into the body fluids of the treated animal.

The rate at which the active ingredients is released from an implant, and hence, the length of time during which the implant remains effective, is controlled with good accuracy by the proper adjustment of the concentration of the compound in the implant, the external area of and amount of carrier in the implant, the external area or the implant, the formulation of the polymer from which the implant is made, the thickness of the wall of hollow implants and the diffusion characteristics of the active or carrier/active solution through the wall of the implant or through specially designed end-plugs of polymer or other membrane forming one or more surfaces of the implant, or by being forced through a porous membrane or aperture by an osmotic pump activated by absorption of body water into an osmotically active component contained in a second compartment of a hollow implant.

Administration of the active ingredient by means of an implant is a further particularly preferred embodiment. Such administration is highly economical and efficacious because a properly designed implant maintains a constant concentration of the compound in the tissues of the host animal, can be designed to supply a compound for several months, and is easily inserted in the animal. No further handling of the animal or concern over the dosage is necessary after implant insertion. The implant may be erodible/soluble and may be left in the animal tissue, or it may be insoluble/non-erodible and suitable for surgical removal after exhaustion of its active ingredient.

A convenient bolus formulation is disclosed in U.S. Pat. No 4,166,107 which may also be adopted for sustained release for the method of this invention.

The formulation of veterinary additives in animal feed is a well known art. It is usual to formulate the compound first as a pre-mix in which the active ingredient is dispersed in a liquid or a particular solid carrier. The pre-mix may conveniently contain from about 1 to about 800 g of compound per kg, depending on the desired concentration in the feed. To prevent hydrolysis or degradation by constituents of the animal feed, the active compound may be formulated in a protective matrix such as gelatin before addition to the pre-mix, and further protected by formulation with suitable preservatives, and antioxidants [e.g., sodium benzoate, parabens, BHT (butylated hydroxytoluene) and BHA (butylated hydroxyanisole)].

Commercial products may be formulated as concentrates, from which the end user will normally employ dilute formulations. The instant compositions may also contain further ingredients such as stabilisers, antioxidants, anti-foams, viscosity regulators, binders, tackifiers, preservatives, as well as other known and active ingredients for obtaining special effects. Materials known from veterinary practice as being suitable for oral, parenteral or implant administration may be employed. A number of examples are cited below.

Suitable carriers may be:
- fillers such as sugars, lactose, sucrose, mannitol or sorbitol, cellulose preparations, and/or calcium phosphates, for example, tricalcium phosphate or calcium hydrogen phosphate,
- binders such as starch pastes, using, for example, corn, wheat, rice, or potato starch, gelatin, tragacanth, methylcellulose and/or if desired,
- disintegrators, such as the above-mentioned starches, carboxymethyl starch cross-linked to polyvinylpyrrolidone, agar, alginic acid, or a salt thereof, such as sodium alginate,
- adjuvants which are flow-regulating agents and lubricants, for example, silica, talc, stearic acid or salts thereof, such as magnesium stearate or calcium stearate, and/or polypropylene glycol.

Dragée cores can be provided with suitable coatings that may be resistant to gastric juices, i.e., concentrated sugar solutions which may include gum arabic, talc, polyvinylpyrrolidone, polyethylene glycol and/or titanium dioxide, or lacquer in suitable organic solvents or solvent mixtures, or for the production of coatings that are resistant to gastric juices, solutions and suitable cellulose preparations, such as acetylcellulose phthalate, or hydroxypropylmethylcellulose phthalate may be used.

Colourings, flavourings or pigments can be added to the tablets of Dragee coatings, for example, for identification purposes or to indicate different doses of active ingredients.

Further orally-administrable preparations are dry filled capsules consisting of gelatin and also soft-sealed capsules consisting of gelatin and plasticizers such as glycerol or sorbitol may be used. The dry filled capsules may contain the active ingredient in the form of a granulate, for example, an admixture with fillers such as lactose, binders, such as starches, and solid lubricants, such as talc or magnesium stearate, and optional stabilisers. In soft capsules, the active ingredients is preferably dissolved or suspended in suitable liquids, such as fatty oils, paraffin oils, or liquid polyethylene glycols, it being possible to also add stabilisers. For example, pharmaceutical preparations for oral administration can be obtained by combining the active ingredient with solid carriers, optionally granulating a resultant mixture, and processing the mixture of granulate, if desired or if necessary, after the addition of suitable adjuncts, to form tablets or Dragee cores.

Especially suitable for parenteral administration are aqueous and oily solutions or suspensions of the active ingredient, such as an oily injection suspension, with a suitable lipophilic solvent or vehicle such as a fatty oil, for example, sesame oil, or synthetic fatty acid esters, for example, ethyloleate, or triglycerides, or aqueous injectable suspensions that contain viscosity increasing substances, for example, sodium carboxymethylcellulose, sorbitol and/or dextran, and, optionally, also stabilisers. The preparations useful for the present invention can be manufactured by conventional methods, such as, for example, mixing, granulating, confectioning, dissolving or lyophilising processes.

Further topical administrations are suitable. Animal tissues comprise various membranes which are selectively permeable and which allow some substances to pass freely. The systemic activity of the compounds according to the present invention has been found especially effective by this way of administration. The penetration into the underlying tissues and/or in the physiological fluids may be obtained from the topical application to physiological membranes like the skin or mucous membranes such as buccal, nasal, rectal or vaginal membranes. Especially suitable for topical administration are aqueous and oily solutions or suspensions of the active ingredients. The solvents for topical applications may be oils for example, sesame oil, synthetic fatty esters for example transesterified triglycerides. The solvents as polyalcohol, glycol, their esters and ether may be used. Other solvents or their combination are particularly suitable like dimethylsulfoxyde, dimethylformamide, dimethylacetamide, glycerol formal, N-methyl-pyrrolidone. Pharmaceutically acceptable hydrophylic diluents, particularly water, may be employed in the compositions to avoid the local tissue irritation.

For topical compositions, the preferred topical carrier is selected from the group consisting of an ether of ethylene or propylene glycol and dimethylsulfoxyde in a ratio of 10/90 to 90/10 and the preferred transdermal carrier is selected from fatty ester eventually transesterified and an ether of ethylene or propylene glycol in a ratio of 10/90 to 90/10.

Especially preferred combinations are the one comprising a parasiticidal effective amount of amidine as defined hereabove, preferably acetamiprid in combination with an effective amount of a compound selected from IGR compounds consisting of pyriproxifen, fenoxycarb, triarathene, thiapronil, hexythiazox, clofentezine, buprofezin, methoprene; IDI (insect development inhibitor) like benzoylurea: lufenuron flufenoxuron, triflumuron, chlorfluazuron, diflubenzuron, flucycloxuron, hexaflumuron, penfluron, teflubenzuron, diafenthuron or cyromazines; phenylpyrazoles: fipronil, tebufenpyrad; phenoxybenzylethers derivatives: ethofenprox, flufenprox, brofenprox; endoparasite control agents: avermectins and their derivatives, preferably milbemycin, ivermectin, milbemycin oxime or moxidectin; benzimidazoles, such as oxfendazole, albendazole, albendazole sulfoxide, fenbendazole, flubendazole, mebendazole, thiabendazole, cambendazole, oxibendazole, parabendazole, cyclobendazole, luxabendazole, thiophanate, netobimin, and triclabenzazole; piperazine and its salts; levamisole; pyrantel; febantel; niclosamide; nitroscanate; praziquantel; natural pyrethrins; synthetic pyrethroids: permethrin, cypermethrin, cyhalothrin, lambdacyhalothrin, deltamethrin tralomethrin, flucythrin, flumethrin, cyfluthrin, allethrin, prallethrin, alphamethrin, bioallethrin, bioresmethrin, cycloprothrin, fenpropathrin, fenfluthrin, trihalomethrin, metrifonate, and their derivatives; organophosphates: diazinon; organochlorides; carbamates: bendiocarb pirimicarb, promecarb, thiodicarb, isoprocarb,carbosulfan; formamidines: as amitraz; or esfenvalerate, such that the active ingredients are transmitted to target parasites via the blood, body fluids or tissue of the warm blooded animal in parasiticidal effective amounts sufficient to protect the animal from multiple parasites.

The instant compositions may therefor comprise an effective dose of amidine, preferably a parasiticidal effective amount of acetamiprid in combinantion with an effective dose of avermectins and their derivatives will be in the range of about 0.01 to about 100 mg/kg, preferably about 0.1 to about 50 mg/kg for milbemycin; about 0.01 to about 10 mg/kg, preferably about 0.025 to about 0.5 mg/kg for ivermectin, about 0.01 to about 100 mg/kg preferably about 0.5 to about 50 mg/kg for milbemycin oximes and about 0.5 to about 100 mcg/kg, preferably about 1.25 to about 50 µg/kg for moxidectin. Especially preferred combinations comprise a parasiticidal effective amount of acetamiprid in combination with an effective amount of a compound selected from the group consisting of milbemycin, milbemycin derivatives, ivermectin, ivermectin derivatives, milbemycin oxime, milbemycin oxime derivatives, moxidectin, and moxidectin derivatives, or mixtures thereof. An especially preferred composition comprises a parasiticidally effective amount of acetamiprid, an effective amount of moxidectin and a pharmaceutically acceptable carrier.

In another preferred embodiment of the instant compositions, they may comprise a parasiticidal effective dose comprising a mixture of from about 0.001 to about 100 mg/kg of acetamiprid and from about 0.5 µg/kg to 1 g/kg body weight of a warm blooded animal of an IGR insecticid.

In another preferred embodiment of the instant compositions, they may comprise a parasiticidal effective dose comprising a mixture of from about 0.001 to about 200 mg/kg of acetamiprid and about 0.5 to about 100 µg/kg of moxidectin to a warm-blooded animal, preferably a dog, cat, cow, sheep, goat, pig, mink, fox, rabbit, chicken, duck or goose.

The following compositions are the most preferred ones :
. composition comprising about 0.2 to about 100 mg/kg of acetamiprid with about 1.25 to about 50 µg/kg avermectin
. composition comprising about 0.2 to about 50 mg/kg acetamiprid and about 1.25 to about 50 mg/kg of moxidectin.

They are administered to a warm-blooded animal, such that parasiticidal effective amounts of the active ingredients are transmitted to the ectoparasites or endoparasites via the blood of the warm-blooded animal.

It is expected that when compositions of the preferred combinations as formulated in examples below are administered to dogs (i) infested with fleas that are maintained in an environment favorable to development of larvae, their pupation and the emergence of new fleas to reinfest the dogs, and (ii) also continually exposed to heartworm infection through the biting of mosquitoes transmitting infective larvae of *D. immitis*,
the test animals will be substantially free of both fleas and heartworms at the end of a 6 months test period,
while similarly maintained control dogs will suffer severe and increasing infestations of both fleas and heartworms.

It is also expected that the ecto- and endoparasiticidal compositions will show a broad protection against fleas, ticks, mosquitoes, lice, mange mites, and biting flies as well as anthelmintic and antiparasitic effects against the metazoan parasites known as Helminthes, especially *Nematoda* and members of the family Filaridae or Setariidae, and the genera *Ancylostoma*, *Uncinaria*, *Toxocara*, *Toxascaris*, *Trichuris*, *Filaroides,* and *Dirofilaria* in dogs and *Nematospiroides*, *Syphacia* and *Aspiculuris* in the rhodentias.

Combinations of acetamiprid with pyriproxifen, milbemycin, ivermectin, milbemycin oxime or moxidectin may also exhibit enhanced or synergistic effects wherein one or more components of the mixtures is more efficacious than the same dose administered alone. The following examples illustrate the invention described herein, but do not limit its scope in any way.

### EXAMPLE 1:

### Tablets (1,000):

Tablets containing 25 mg of active ingredient, for example, acetamiprid, can be manufactured as follows (for 1,000 tablets):

| CONSTITUENTS | AMOUNT |
|---|---|
| Active ingredient | 25.0 g |
| Lactose | 100.7 g |
| Wheat Starch | 7.5 g |
| Polyethylene glycol (mol. wt. 6,000) | 5.0 g |
| Talc | 5.0 g |
| Magnesium stearate | 1.8 g |
| Demineralized water | q.s. |

All of the solid ingredients are first forced through a sieve having a mesh of 0.6 mm, then the active ingredient, the lactose, the talc, the magnesium stearate, and half the starch, are mixed. The other half of the starch is suspended in 40 ml. of water and the suspension is added to a boiling solution of the polyethylene glycol in 100 ml of water. The resulting starch base is added to the main batch and the mixture, if necessary, with the addition of water, is granulated. The granulate is dried overnight at 35°C, forced through a sieve having a mesh width of 1.2 mm and pressed to form tablets.

### EXAMPLE 2:

### Tablets (1,000):

Tablets containing 20 mg of active ingredient, for example, acetamiprid, can be manufactured as follows (for 1,000 tablets):

| CONSTITUENTS | AMOUNTS |
|---|---|
| Active ingredient | 20.0 g |
| Lactose | 290.8 g |
| Talc | 200.0 g |
| Potato Starch | 274.7 g |
| Stearic Acid | 10.0 g |
| Magnesium Stearate | 2.5 g |
| Colloidal Silica | 30.0 g |
| Ethanol | q.s. |

The mixture of the active ingredient, the lactose and 194.70 g of potato starch, is moistened with an ethanolic solution of the stearic acid and granulated through a sieve. After drying the remainder of the potato starch, talc, magnesium stearate, and colloidal silica are added mixed to the mixtures and pressed to form 0.1 g tablets which, if desired, can be provided with breaking grooves for finer adjustment of the dosage.

### EXAMPLE 3:

### Capsules:

Capsules containing 25 mg of active ingredient, for example, acetamiprid, can be manufactured as follows:

| CONSTITUENTS | AMOUNTS |
|---|---|
| Active Ingredient | 25.0 g |
| Lactose | 249.0 g |
| Gelatin | 2.0 g |
| Corn Starch | 10.0 g |
| Talc | 15.0 g |
| Water | q.s. |

The active ingredient is mixed with the lactose and the mixture is moistened uniformly with an aqueous solution of the gelatin and granulated through a sieve having a mesh width of 1.2-1.5 mm. The granulate is then mixed the dried cornstarch and the talc and introduced in portions of 300 mg into hard gelatin capsules (size 1).

### EXAMPLE 4:

### Pre-mix (feed additive):

Twenty-five hundredths (0.25) parts by weight of active ingredients, for example, acetamiprid, and 4.75 parts of secondary calcium phosphate, or China clay, aerosil or carbonate or lime or homogeneously mixed with 95 parts of an animal feed.

### EXAMPLE 5:

### Emulsifiable concentrate :

Twenty (20) parts of active ingredient, for example, acetamiprid, are mixed with 20 parts of emulsifier (e.g., a mixture of alkylarylpolyglycol ether with alkylarylsulphonates), and 60 parts of solvent until the solution is completely homogenous. By diluting this concentrate with water, it is possible to obtain an emulsion of a desired concentration.

### EXAMPLE 6:

### Soluble powder:

Twenty-five (25) parts of active ingredient, for example, acetamiprid; 1 part sodium lauryl sulfate; 3 parts colloidal silica; and 71 parts urea are mixed and the mixture is finally ground in a suitable mill.

### EXAMPLE 7:

Gels can be manufactured as follows:

| CONSTITUENT | AMOUNT |
|---|---|
| Active Ingredient | 0.5 g |
| Cod Liver Oil | 12.5 g |
| Corn Oil | 124.5 g |
| Beef Peptone | 2.5 g |
| Glucose | 12.5 g |
| Sodium Benzoate | 5.0 g |
| Methyl Cellulose | 12.5 g |
| Molasses | 5.0 g |
| Demineralized Water | 75.0 g |

The active ingredient, for example, acetamiprid, is dissolved in the oil phase. Glucose, peptone, sodium benzoate and molasses are dissolved in the demineralized water heated to 60°C. into which the methyl cellulose is blended to form a gel, which is then blended with the oil phase. The gel is filled into flexible plastic squeeze tubes which are crimp sealed and capped. Alternatively, the gel may be filled into ^{〈〈}dial-a-dose^{〉〉} syringes graduated to supply the desired daily dose, for instance 1 g/10 kg body weight. This basic daily oral dose form gel may be improved by addition of vitamins (e.g., A, D, E and the B group), minerals, trace elements and essential fatty acids, to provide an easy-to-administer, complete nutritional supplement for pet animals that also provides effective ectoparasite control. The proportions of ingredients set out above provide 2.0 mg of active ingredient per gram of gel.

### EXAMPLE 8:

### Dietary supplement:

| CONSTITUENT | AMOUNT |
|---|---|
| Active Ingredient | 1.0 g |
| Fish Oil (Omega 3) | 80.0 g |
| Borage Seed Oil (gamma Linolenic Acid) | 20.0 g |
| Sunflower Seed Oil | 847.9 g |
| Chicken Flavour | 5.0 g |
| Butyrated Hydroxytoluene | 1.0 g |
| Propylene Glycol | 20.0 g |
| Zinc Sulfate | 1.0 g |
| Tocopherol Acetate | 3.0 g |
| Inositol | 0.5 g |
| Pyridoxime Hydrochloride | 0.1 g |
| Vitamin A Palmitate | 0.1 g |
| Biotin | 0.1 g |
| Propyl-Methyl Parabens | 0.3 g |

All ingredients are dissolved/blended in the oil phase. The liquid supplement is filled into flexible plastic squeeze ^{〈〈}tip-and-measure^{〉〉}, litre bottles which are capped. Daily dosage as a liquid food additive for dogs and cats is as appropriate to body weight and desired daily dose rate (mg active compound/kg body weight). Administration of dermatologically-active essential fatty acid/vitamine/zinc supplements are especially beneficial for pet animals suffering from dermatitis caused by exposure to infestations of ectoparasites. The proportions of ingredients set out above provides, 1 mg active ingredient for example, acetamiprid, per gram of dietary supplement.

### EXAMPLE 9:

### Monolithic 150 mg Implant:

Active ingredient (30.0 mg), for example acetamiprid, is mixed with the prepared methacrylate hydrophilic copolymer powder (30.0 mg) to which is added 90.0 mg of elastomeric silicone composite, for instance hydroxy-hydrogen-poly (dimethylsiloxane) and methyltriacetoxysiloxane cross linking agent. The mixture is reacted at ambient temperature in a mold for 12 hours. Implants are sterilised by gamma radiation or by gassing with ethylene oxide after packaging. Implants are inserted subcutaneously either by trochar or by minor surgical procedure. Multiple implants may be inserted simultaneously depending on the animal's weight, on the known release rate of active ingredient and on the desired frequency of reimplantation.

### EXAMPLE 10:

Transdermal Gel may be manufactured as follows:

| CONSTITUENT | AMOUNT |
|---|---|
| Active Ingredient | 12.5 g |
| Corn Oil | 12.5 g |
| Dimethylsulfoxide | 12.5 g |
| Butyrated Hydroxytoluene | 1.3 g |
| Methyl Cellulose | 5.0 g |
| Demineralized Water | 206.3 g |

The active ingredient is dissolved in the oil phase to which the other excipients are added and the mixture blended to a smooth gel of viscosity 1000-1200 centipoise. The gel is filled in 2 ml aliquots into unit dose flexible plastic squeeze tubes which are heat sealed. Alternatively, the gel may be filled into ^{〈〈}dial-a-dose^{〉〉}, multi-dose syringes graduated and adjustable to supply, at the appropriate dose rate, the desired monthly dose, for topical application directly on to the skin of the animal's back. The proportions of ingredients set out above provides 50 mg of active ingredient per gram of transdermal gel.
Other biocidally-active ingredients or agents which are inert towards the active ingredients and acceptable to the animals to be treated or mineral salts or vitamins can be admixed with the compositions above described. In a manner analogous to that described in the formulation Examples 1 to 10, it is possible to manufacture corresponding preparations containing any compound selected from the group of compounds as defined above, and in combination with one or more additional active ingredients.

### EXAMPLE 11

### Pour-on, Spot-on formulation of acetamiprid

a ) the active ingredient acetamiprid (10 g) is dissolved in 50 ml of ethyl lactate and an alkylether of the ethylene or propylene glycol is added with stirring to complete 100 ml. The solution is stable and can be applied directly to the animal.
b ) the active ingredient acetamiprid (10 g) is dissolved in 20 ml of dimethylsulfoxyde and 40 ml of alkylether of the ethylene or propylene glycol; after complete solubilization; then the volume is adjusted to 100 ml with alkylether.
c ) the active ingredient acetamiprid (10 g) is dissolved in 50 ml of dimethylsulfoxyde, 40 ml of an alkylether of the ethylene or propylene glycol is added; then the volume is adjusted to 100 ml with DMSO.

### EXAMPLE 12

### Pour-on, Spot-on formulation of an association

a ) the active ingredient acetamiprid (10 g) is dissolved in 50 ml of ethyl lactate; 1 g of pyriproxifen is added and after solubilization, alkylether of the ethylene or propylene glycol is added with stirring to adjust to 100 ml. The solution is stable and can directly be applied to the animal.
b ) the active ingredient acetamiprid (10 g) is dissolved in 20 ml of dimethylsulfoxyde and 40 ml of alkylether of the ethylene or propylene glycol; 1 g of pyriproxifen is added and after solubilization, the volume is adjusted to 100 ml with alkylether.
c ) the active ingredient acetamiprid (10 g) is dissolved in 50 ml of dimethylsulfoxyde, 40 ml of alkylether of the ethylene or propylene glycol is added as well as 1 g of pyriproxifen; the volume is then adjusted to 100 ml with DMSO.
   The alkylether of the ethylene or propylene glycol may be, for example, Dowanol®.

### EXAMPLE 13

### Foam preparation of a composition containing acetamiprid and IGR

The preparation is made as following:
- Acetamiprid: 0.8 %
- Ethanol: 10 %
- Methylol: 10 %
- Pyriproxifen: 0.012 %
- Ceteareth (ether PEG cetearyl alcohol): 2 %
- POE 35 of ricin oil: 1 %
- Cocamide DEA: 0.2 %
- Denatonium benzaote: 0.025 %
- Water: adjusted to 100 ml

### EXAMPLE 14

### Spray preparation

- Acetamiprid: 0.8 %
- Ethanol: 15 %
- Denatonium benzoate: 0.025 %
- Ceteareth 25: 2.0 %
- POE 35 ricin oil: 1 %
- Sodium lauroyl sarcosinate: 2 %
- Cocamide DEA: 0.2 %
- Water: adjusted to 100 ml
- butane /* Propane: propulsive vector

### EXAMPLE 15

### Injectable preparation

A solution is prepared by dissolving 10 g of acetamiprid in Q.S.P. Glycerol formal to complete to 100 ml ; thereafter, the solution is sterilised by a convenient system.

### EXAMPLE 16

### Flea control through oral administration of active ingredient.

Acetamiprid was formulated in a petroleum base paste containing 10% by weight active ingredient, packaged in a 〈〈 dial-a-dose 〉〉 syringe for oral administration. The active ingredient load of 10% was designed to provide a nominal dose of 10 mg/kg body weight of three treated dogs when administered at the dose of 0,1 ml of paste per kilogram body weight. The test subjects were good flea hosts, docile and amenable to oral dosing. The test subjects were gang housed by groups of three, with water and food ad lib. Three additional dogs were maintained in the same fashion as controls. The dogs were experimentally infested with newly emerged adult fleas. (*C. Felis*) on days prior to treatment and subsequently on a weekly basis. The percentage reduction of adult flea population was determined by combing the animal, comparing the number of fleas persisting on treated animals to control, at 1 and 3 days post treatment and subsequent reinfestation cycle. The results are presented below :

**Table 1**

| Acetamiprid Oral Dosage Administered | | | | | |
|---|---|---|---|---|---|
| | | Paste | Paste | Acetamiprid | Dose mg/kg |
| Dog N° | Weight (kg ) | grams | milliliters | milligrams | mg/kg |
| 3211 | 10.70 | 1 | 0.98 | 98.0 | 9.2 |
| 3226 | 16.05 | 1.5 | 1.47 | 147.0 | 9.2 |
| 3227 | 15.70 | 1.6 | 1.57 | 156.8 | 10.0 |
| 3161 | 19.15 | - | - | - | - |
| 3209 | 15.55 | - | - | - | - |
| 3214 | 8.95 | - | - | - | - |

**Table 2**

| Acetamiprid Oral - Systemic effect on Adult Fleas Post Treatment Flea Population Reduction Day post Treatment | | | | | | | |
|---|---|---|---|---|---|---|---|
| Dog N° | Group | D1 | D1 | D2 | D2 | D3 | D3 |
| | | Flea Count | Efficacy | Flea Count | Efficacy | Flea Count | Efficacy |
| 3211 | treated | 31 | 13 % | 19 | 42 % | 8 | 76 % |
| 3226 | treated | 21 | 41 % | 12 | 66 % | 6 | 82 % |
| 3227 | treated | 28 | 21 % | 17 | 51 % | 4 | 88 % |
| **Mean** | **treated** | **26.7** | **25 %** | **16.0** | **54 %** | **6.0** | **82 %** |
| 3161 | control | 39 | | 41 | | 40 | |
| 3209 | control | 37 | | 35 | | 31 | |
| 3214 | control | 31 | | 29 | | 30 | |
| **Mean** | **control** | **35.7** | | **35.0** | | **33.7** | |

**Table 3**

| Acetamiprid Oral Systemic Efficacy on Adult Fleas on Reinfestation Day Post Treatment/Day Post Reinfestation | | | | | | | | | |
|---|---|---|---|---|---|---|---|---|---|
| Dog N° | Group | D7/D1 | D7/D1 | D9/D3 | D9/D3 | D14/D1 | D14/D1 | D16/D3 | D16/D3 |
| | | Fleas | Efficacy | Fleas | Efficacy | Fleas | Efficacy | Fleas | Efficacy |
| 3211 | Treated | 29 | 42 % | 11 | 75 % | 16 | 69 % | 18 | 67 % |
| 3226 | Treated | 23 | 54 % | 16 | 63 % | 47 | 10 % | 53 | 2 % |
| 3227 | Treated | 21 | 58 % | 11 | 75 % | 28 | 46 % | 32 | 41 % |
| **Mean** | **Treated** | **24.3** | **51 %** | **12.7** | **71 %** | **30.3** | **42 %** | **34.3** | **36 %** |
| 3161 | Control | 43 | | 41 | | 63 | | 68 | |
| 3209 | Control | 47 | | 44 | | 43 | | 47 | |
| 3214 | Control | 59 | | 46 | | 51 | | 47 | |
| **Mean** | **Control** | **49.7** | | **43.7** | | **52.3** | | **54.0** | |

Comparable results are obtained when the active ingredient is systemically administered to other host species as in Example 17 below, or when other active compounds are substituted to acetamiprid, or other target parasites are substituted to fleas, as in Example 18. Additional active compounds may also be added to the mixture to produce systemic protection against multiple target parasites or to gain an increased control of a single target parasite as in improved flea control.

### EXAMPLE 17:

Flea Control Through Injectable Administration of the Active Ingredient. Acetamiprid was administred to four cats as a 10% injectable solution for subcutaneous administration. The active ingredient load of 10% was designed to provide a nominal dose of 5 mg/kg for each of the treated cats when administered as a dose of 0.05ml/kg body weight. The test subjects were good flea hosts, individually caged, food and water ad lib. Four additional cats were maintained as control. The cats were experimentally infested with newly emerged adult fleas (*C. felis*) on the day prior to treatment, and subsequently reinfested by combing animal, comparing the number of adults fleas persisting on treated animals to control at 1 and 3 days following treatment or reinfestation. The data below show partial or complete reduction on the animals systemically treated with the compound.

**Table 4**

| Acetamiprid Injectable systemic Efficacy on Adult Fleas Dosage Administred - Injectable solution 10% Application Rate of 0.05 ml/kg body weight | | | | |
|---|---|---|---|---|
| Cat N°. | Cat Weight | Injected dose | Acetamiprid | Ratio |
| | kg | ml | mg | mg/kg |
| 2344 | 3.85 | 0.2 | 20.0 | 5.2 |
| 2342 | 2.85 | 0.15 | 15 | 5.3 |
| 4860 | 3.25 | 0.15 | 15.0 | 4.6 |
| 2324 | 4.40 | 0.22 | 22.0 | 5.0 |
| 2339 | 6.45 | | | |
| 2310 | 2.35 | | | |
| 4825 | 2.60 | | | |
| 2312 | 4.25 | | | |

**Table 5**

| Acetamiprid Injectable - Systemic Efficacy on Adult Fleas Post Treatment Flea Population Day Post Treatment/Post Reinfestation | | | | | | | | | |
|---|---|---|---|---|---|---|---|---|---|
| Cat N° | Group | D1 | D1 | D3 | D3 | D7/D1 | D7/D1 | D9/D1 | D9/D1 |
| | | Fleas | Efficacy | Fleas | Efficacy | Fleas | Efficacy | Fleas | Efficacy |
| 2344 | treated | 0 | 100 % | 0 | 100 % | 21 | 68 % | 25 | 51 % |
| 2342 | treated | 0 | 100 % | 0 | 100 % | 36 | 41 % | 56 | - 9 % |
| 4860 | treated | 0 | 100 % | 0 | 100 % | 28 | 57 % | 44 | 15 % |
| 2324 | treated | 0 | 100 % | 0 | 100 % | 7 | 89 % | 27 | 48 % |
| Mean | treated | 0.0 | 100 % | 0.0 | 100 % | 23.0 | 65 % | 38.0 | 26 % |
| 3161 | control | 62 | | 63 | | 78 | | 82 | |
| 3209 | control | 67 | | 59 | | 54 | | 32 | |
| 4852 | control | 64 | | 65 | | 69 | | 54 | |
| 3214 | control | 62 | | 61 | | 62 | | 38 | |
| Mean | control | 63.8 | | 62.0 | | 65.8 | | 51.5 | |

### EXAMPLE 18:

Tick Control Through the Oral Administration of an Active Compound. In the experiment of Example 10 above, the Dogs were also experimentally infested with newly emerged Brown Dog Ticks (*R. sanguineous*), at the same time as the flea infestations. The percentage reduction of tick population was evaluated as in Examples 10 and 16 above.

**Table 6**

| Acetamiprid Oral Systemic Efficacy on Adult Ticks Post Treatment tick Population Reduction | | | | | | | |
|---|---|---|---|---|---|---|---|
| Dog N° | Group | D1 | D1 | D2 | D2 | D3 | D3 |
| | | Tick Count | Efficacy | Tick Count | Efficacy | Tick Count | Efficacy |
| 3211 | treated | 9 | 45 % | 6 | 63 % | 2 | 88 % |
| 3226 | treated | 12 | 27 % | 6 | 63 % | 0 | 100 % |
| 3227 | treated | 16 | 2 % | 12 | 27 % | 4 | 76 % |
| **Mean** | **treated** | **12.3** | **24 %** | **8.0** | **51 %** | **2.0** | **88 %** |
| 3161 | control | 13 | | 13 | | 91 | |
| 3209 | control | 19 | | 18 | | 11 | |
| 3214 | control | 17 | | 18 | | 19 | |
| **Mean** | **control** | **16.3** | | **16.3** | | **16.3** | |

**Table 7**

| Acetamiprid Oral Systemic Efficacy on Adult Ticks on Reinfestation Day Post Treatment/Day Post Reinfestation | | | | | | | | | |
|---|---|---|---|---|---|---|---|---|---|
| Dog N° | Group | D7/D1 | D7/D1 | D9/D3 | D9/D3 | D14/D1 | D14/D1 | D16/D3 | D16/D3 |
| | | Ticks | Efficacy | Ticks | Efficacy | Ticks | Efficacy | Ticks | Efficacy |
| 3211 | Treated | 9 | 56 % | 0 | 100 % | 6 | 49 % | 7 | 50 % |
| 3226 | Treated | 9 | 56 % | 6 | 61 % | 12 | - 3 % | 23 | - 64 % |
| 3227 | Treated | 11 | 47 % | 4 | 74 % | 10 | 14 % | 15 | - 7 % |
| **Mean** | **Treated** | **9.7** | **53 %** | **3.3** | **78 %** | **9.3** | **20 %** | **15.0** | **- 7 %** |
| 3161 | Control | 19 | | 14 | | 14 | | 23 | |
| 3209 | Control | 24 | | 14 | | 8 | | 10 | |
| 3214 | Control | 19 | | 18 | | 13 | | 9 | |
| **Mean** | **Control** | **20.7** | | **15.3** | | **11.7** | | **14.0** | |

### EXAMPLE 19:

Flea control through the pour-on administration of the active ingredient Acetamiprid was administered to 6 dogs as a spot-on formulation by topical administration.
The active ingredient load of 10% was designed to provide a nominal dose of 30 mg/kg for each of the treated dogs when administered as a dose of 0,3 ml/kg body weight. The tests subjects were good flea hosts, individually caged, food and water ad libitum. Six additionnal dogs were maintened as control. The dogs were experimentally infested with newly emerged adult fleas (*G. felis*) on the day prior to treatment, and subsequently reinfested by combining animal, comparing the number of adults fleas persisting on treated animals to control at 1, 3, 7, 14, 21, 28, 35, 42, 49, days following treatment.
The data below show complete reduction on the animals systemically treated with the compound.

| Day | 1 | 3 | 7 | 14 | 21 | 28 | 35 | 42 | 49 |
|---|---|---|---|---|---|---|---|---|---|
| Protec. | 97% | 100% | 100% | 100% | 100% | 97% | 93% | 90% | 80% |

### EXAMPLE 20:

Tick control through spot-on administration of the active ingredient.
Acetamiprid was administered to 6 dogs as a 10% spot-on formulation by topical administration.
The active ingredient load of 10% was designed to provide a nominal dose of 30 mg/kg for each of the treated dogs when administered as a dose of 0,3 ml/kg body weight. The tests subjects were good tick hosts, individually caged, food and water ad libitum. Six additionnal dogs were maintened as control. The dogs were experimentally infested with brown dog Ticks (*Rhipicephalus sanguineus*) unengorged adults. Comparing the number of on live ticks on treated animals to control at 1, 3, 7, 9 days, show a good curative effect.
The preceding examples illustrate formulations and demonstrate the efficacy of the instant compounds and compositions against common parasites of pet animals. Similar results will be obtained when other members of the claimed genus are substituted in similar experiments or when given to other warm blooded animals against the same or other target parasites.
As is apparent from the foregoing description, the invention is in no way limited to those modes of execution, embodiments and modes of application which have now been described more explicitly; on the contrary, it encompasses all the variants thereof which may occur to those skilled in the art, without deviating from the framework or the scope of the present invention.

## Claims

1. An amidine of the formula I: 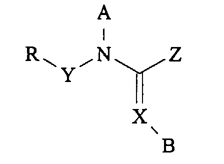
wherein R represents an optionally substituted 5 or 6 member aromatic ring containing at least one nitrogen atom;
Y represents an optionally substituted C₁-C₃ alkylene or alkylidene;
A represents a hydrogen, a carbamoyl, a mono or di C₁₋₅ alkyl carbamoyl, a thiocarbamoyl, a mono or di C₁₋₅ alkylthiocarbamoyl, a sulfamoyl, a mono or di C₁₋₅ alkylsulfamoyl, an optionally substituted C₁₋₅ alkyl, an optionally substituted C₂₋₅ alkenyl, an optionally substituted C₂₋₅ alkynyl, an optionally substituted C₃₋₈ cycloalkyl, an optionally substituted C₃₋₈ cycloalkenyl, an optionally substituted aryl or -D-R₂, where D is O, S(O)_{n'} CO, CS or CO₂; n is 0, 1, or 2, and R₂ is a hydrogen, an optionally substituted C₁₋₅ alkyl, an optionally substituted C₂₋₅ alkenyl, an optionally substituted C₂₋₅ alkynyl, an optionally substituted C₃₋₈ cycloalkyl, an optionally substituted C₃₋₈ cycloalkenyl, an optionally substituted aryl;
Z represents a hydrogen, an optionally substituted C₁₋₅ alkyl, an optionally substituted C₂₋₅ alkenyl, an optionally substituted C₂₋₅ alkynyl, an optionally substituted C₃₋₈ cycloalkyl, an optionally substituted C₃₋₈ cycloalkenyl;
B represents a cyano or a nitro; and
X represents CH or N; or its salt
for use in treating by systemic administration, both ecto and/or endo parasitic diseases, in warm blooded animals.

2. The amidine according to claim 1, wherein the compound is N-[(6-halo-3-pyridinyl)C₁₋₃ alkyl)]-N-cyano-N-C₁₋₃ alkylethaneimidamide.

3. The amidine according to claim 1, wherein the compound is N-[6-chloro-3-pyridinyl)methyl)]-N-cyano-N-methylethaneimidamide.

4. A composition containing an effective parasiticidal amount of at least one amidine according to claims 1 to 3 in combination with at least one carrier, diluent or excipient therefor and eventually an effective paarasiticidal amount of another parasiticidal compound, for systemic use in treating both ecto and endo parasitic diseases.

5. The composition according to claim 4, wherein it is given by oral administration.

6. The composition according to claim 4, wherein it is given by parenteral administration.

7. The composition according to claim 4, wherein it is given by topical administration and includes a transdermal carrier.

8. The composition according to claim 7, wherein the topical carrier is selected from the group consisting of an ether of ethylene or propylene glycol and dimethylsulfoxyde in a ratio of 10/90 to 90/10.

9. The composition according to claim 7, wherein the transdermal carrier is selected from fatty ester eventually transesterified and an ether of ethylene or propylene glycol in a ratio of 10/90 to 90/10.

10. The composition according to claim 7, wherein it is in the form of a shampoo or a foam.

11. The composition according to claim 4, wherein it is in the form of an implant.

12. The composition according to claim 4, wherein said amidine is supplied in a dose of from 0.0001mg/kg to 1,000mg/kg of body weight, preferably in a dose from 0.001 mg/kg to 100 mg/kg of body weight and most preferably in a dose from 0.01 mg/kg to 10 mg/kg.

13. The composition according to claim 4, wherein the other parasiticidal compound is selected from IGR compounds consisting of pyriproxifen, fenoxycarb, triarathene, thiapronil, hexythiazox, clofentezine, buprofezin, methoprene ; IDI (insect development inhibitor) like benzoylurea: lufenuron flufenoxuron, triflumuron, chlorfluazuron, diflubenzuron, flucycloxuron, hexaflumuron, penfluron, teflubenzuron, diafenthuron or cyromazines ; phenylpyrazoles: fipronil, tebufenpyrad; phenoxybenzylethers derivatives: ethofenprox, flufenprox, brofenprox ; endoparasite control agents: avermectins and their derivatives, preferably milbemycin, ivermectin, milbemycin oxime, abamectine, doramectine or moxidectin; benzimidazoles, such as oxfendazole, albendazole, albendazole sulfoxide, fenbendazole, flubendazole, mebendazole, thiabendazole, cambendazole, oxibendazole, parabendazole, cyclobendazole, luxabendazole, thiophanate, netobimin, and triclabenzazole; piperazine and its salts; levamisole; pyrantel; febantel; niclosamide; nitroscanate; praziquantel; natural pyrethrins; synthetic pyrethroids: permethrin, cypermethrin, cyhalothrin, lambdacyhalothrin, deltamethrin tralomethrin, flucythrin, flumethrin, cyfluthrin, allethrin, prallethrin, alphamethrin, bioallethrin, bioresmethrin, cycloprothrin, fenpropathrin, fenfluthrin, trihalomethrin, metrifonate, and their derivatives; organophosphates: diazinon; organochlorides; carbamates: bendiocarb pirimicarb, promecarb, thiodicarb, isoprocarb,carbosulfan; formamidines: as amitraz; or esfenvalerate.

14. The composition according to claim 13, wherein said second parasiticidal compound is supplied in a dose of from 0.0001 mg/kg to 1000 mg/kg, preferably in a dose of form 0.001 mg/kg to 100 mg/kg, most preferably in a dose from 0.01 mg/kg to 10 mg/kg.
